# EUROPEAN PATENT APPLICATION

(11) **EP 1 614 407 A2**
(43) Date of publication of application: **11.01.2006**
(21) Application number: 05013681.1
(22) Date of filing: 24.06.2005
(51) Int. Cl.: A61F 13/496, A61F 13/15

(54) **Pants type diaper and corresponding manufacturing process**

(30) Priority: 07.07.2004 IT to20040460
(71) Applicant: Fameccanica. Data S.p.A., 66020 Sambuceto di S. Giovanni Teatino (Chieti) (IT)
(72) Inventor: Pasqualoni, Paolo, 66020 Sambuceto di San Giovanni Teatino (IT)
(74) Representative: Bosotti, Luciano

(57) **Abstract**

An absorbent hygienic-sanitary product (10), which can be worn as a pantlike garment, comprises:
- a central body (12), which is at least partially absorbent, defining the crotch portion of the product (10); the central body (12) having opposite ends each with two opposite side edges (12a) and a terminal edge (12b); and
- at at least one of said opposite ends of said central body (12):
   - two elasticated panels (16) extending from either of the aforesaid side edges (12a) so as to define, at the corresponding ends of the central body (12), a respective portion of waist band of the product (10); and
   - an elasticated band (18) extending adjacent to the aforesaid terminal edge (12b) and the aforesaid elasticated panels (16) so as to define, at each corresponding end of the central body (12), a respective elasticated waist-band portion (18) of the product (10).

## Description

The present invention relates to absorbent sanitary products that can be worn as pants.

For many years, the solution adopted in a practically uniform way by all the producers of the sector has been that of making said products in the form of elements shaped according to a general hourglass conformation, with a central body in which there is located an absorbent composite structure that is designed to collect the body fluids evacuated and two end parts, a front one and a rear one, which extend laterally. The product is worn by bestowing thereon a general U-shaped conformation and bringing the median stretch of the central body (the so-called "crotch portion") to extend between the legs of the user. The end parts are extended around the waist of the user and are connected to one another at the homologous side edges, for example via adhesive tabs, which can normally be repositioned, or similar fastening elements in such a way as to be able to refasten the product around the body of the user.

These products have traditionally been manufactured and sold in an open condition, i.e., leaving it to the person who applies the product the task of setting it around the body of the user and refastening it according to a general pant conformation in the terms referred to previously.

Over the last few years, there has emerged a renewed interest in diapers (nappy-pants) of the type commonly referred to as "training pants". These are diapers of the type illustrated, for example, in US-A-4 610 680, which are packaged and sold in a closed condition. When the product is taken out of the pack, it has a conformation that substantially resembles that of a pair of pants. It is put on by sliding it over the legs of the user according to criteria basically similar to the ones adopted for putting on pants.

Absorbent products of the training-pant type are roughly classifiable in two major categories, identified by the criteria of production.

In particular, these are commonly referred to as either machine-direction (MD) products or cross-direction (CD) products.

The products of the first type substantially resemble, both as regards the structure and as regards the criteria of fabrication, to diapers of a traditional type, which are designed to be sold open. Basically, all the operations that lead to the fabrication of the product are carried out whilst the products are being fed "lengthwise", i.e., with the parts that are to form the front region and the rear region of the product as worn arranged in sequence in the direction of feed.

The products of the second type mentioned previously (i.e., the cross-direction products, obtained by feeding the products set crosswise with respect to the direction of feed) are particularly widespread on Far East markets and are characterized by front and rear areas with very extensive elastication.

Usually, the aforesaid elasticating function is achieved by associating to the structure of the product elastic materials made of threads of yarn and/or tabs, such as Lycra and/or rubber. It is precisely on account of this elastication of the front and rear areas formed by elastic threads that it is practically imperative to form the chassis of the product by operating crosswise.

According to more traditional solutions, the cross-direction mode of fabrication was applied also to the absorbent part (the so-called "core") contained in the central body of the product.

The development of the product, with the increase in the amount of superabsorbent material (SAP) and the reduction in the content of cellulose imposed by the need to improve the fit of these products, has, however, rendered necessary introduction of further elements, such as for example the so-called "cuffs" or elasticated sides, designed to improve the characteristics of side seal of the product in regard to the evacuated body fluids. The application of these additional elements, which is simple and can be implemented using techniques that are consolidated in processes of a machine-direction type, usually proves somewhat complicated and such as to give rise to results that are far from reliable in the case of products obtained using the cross-direction technique.

For this reason, solutions have been proposed in which, in the framework of a process of production that is basically of the cross-direction type, an insert is first fabricated, thus obtaining all the particular characteristics of the central body of the product operating according to the machine-direction mode. The insert is then cut, rotated through 90°, and applied on the chassis of the product, operating crosswise. It may be stated that the vast majority of machines operating with the cross-direction technique currently in use exploit this solution or dual configuration.

Reasoning once again in very general terms, it may be stated that the products of the machine-direction type are appreciated in particular on account of the possibility of providing them with elastic panels that are very wide on the hips. In this way, it is possible to bestow upon the product better qualities of fit, linked to the fact that these products swathe the body of the user in a particularly comfortable way.

On the other hand, one of the most appreciated aspects of the cross-direction products lies in the presence of the elastication of the front and rear regions.

A drawback lamented is, however, represented by the fact that this elastication extends also into the central areas of the front and rear regions of the product, where the ends of the insert containing the absorbent core of the product are located.

To limit this drawback there is known the solution of making the elastication of the front and rear parts of the cross-direction products without gluing the elastics in a position corresponding to the insert. These elastics are then cut after laminating them with the remaining parts (typically the so-called "backsheet" and possible covering layers associated thereto).

The operation of cutting the elastics inevitably also leads to cutting into the layers, such as the backsheet, to which said elastics are associated. The fact of cutting into the backsheet (i.e., in practice, the outer sheet of the product) does not usually create problems from the standpoint of liquid tightness. The insert containing the absorbent core is in fact provided with a film that functions as impermeable barrier. However, the fact of cutting into the backsheet leads to the formation of more or less small tears, with negative effects both on the appearance of the product and on the capacity of the backsheet to withstand the stresses to which the nappy is normally subjected.

The purpose of the present invention is to provide a solution that can integrate within it the intrinsic advantages of the solutions described previously, at the same time preventing the drawbacks outlined above.

In particular, the purpose of the present invention is to provide a product that can be obtained according to modalities that basically resemble the cross-direction mode of production, giving rise, however, to an end product the characteristics of which basically resemble those of a product obtained using machine-direction technology.

According to the present invention, said purpose is achieved thanks to a hygienic-sanitary product having the characteristics recalled specifically in the ensuing claims. The invention relates also to the corresponding method of fabrication.

The invention will now be described purely by way of non-limiting example, with reference to the annexed plate of drawings, in which:
- Figure 1 is a general view of a sanitary product that can be obtained according to the invention;
- Figure 2 is a view of the product of Figure 1 represented in an open and extended condition, with the purpose of illustrating more fully its structural characteristics;
- Figures 3 to 6, where Figure 5 is a cross-sectional view according to the line V-V of Figure 3, are various views of equipment that can be used for manufacturing the product represented in Figures 1 and 2;
- Figure 7 is a general perspective view of a chain of products in the course of processing, basically at the point indicated by the arrow VII of Figure 6; and
- Figures 8 and 9 are schematic illustrations of a variant embodiment of the solution according to the invention.

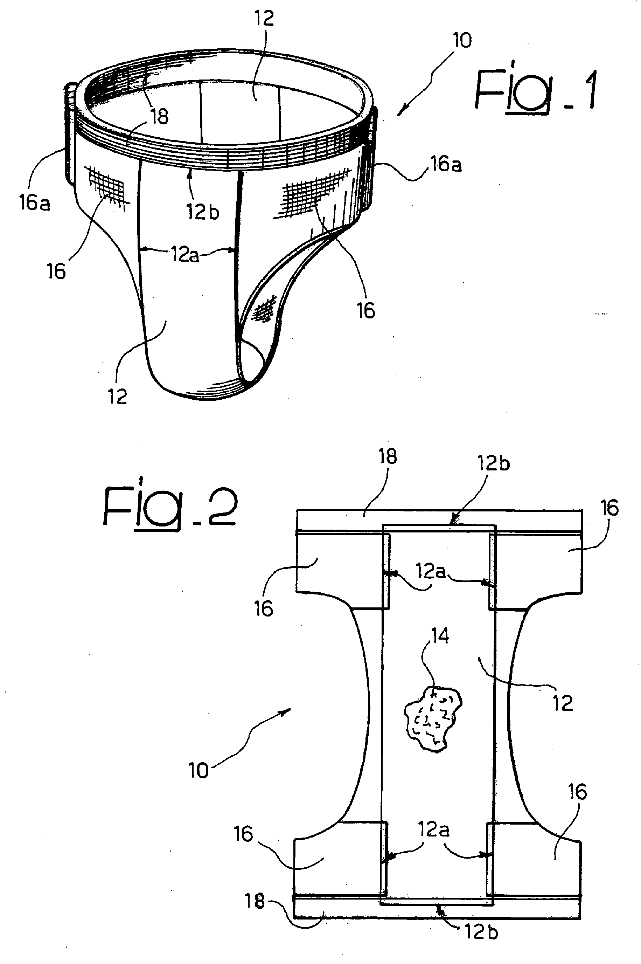

In Figure 1, the reference number 10 indicates as a whole a sanitary product which can be worn as a pantlike garment of the type commonly referred to as "training pant".

Essentially, the product 10 consists of a central body 12 designed to be applied on the body of the user by bestowing upon the product a general U shape. In the body 12 there is located the absorbent core 14, which is designed to absorb the body fluids evacuated. There are then present elasticated side panels, designated by the reference number 16, which extend from the central body 12 so as to bestow upon the product 10 (viewed ideally in the open and distended condition, as represented in Figure 2) the typical hourglass conformation.

The elasticated side panels 16 are provided with homologous distal edges 16a designed to be connected to one another via a line of welds that enable fastening of the product 10, bestowing thereon the desired pant conformation.

The representation of the product 10 provided in Figure 1 is of a schematic nature and is intended to highlight the fact that the solution described herein can be applied to a wide variety of possible types of embodiment of the product 10.

For a more detailed illustration of the characteristics of the product 10 (for example, as regards the presence of shaped edges 13 - usually provided on the backsheet of the product -delineating the borders of the openings for the legs of the user and again on account of the presence of the so-called "cuffs" or elasticated borders 15 arranged along the sides of the absorbent core 14 with the function of providing lateral containment of body fluids) the reader is referred to the extensive literature existing on the subject.

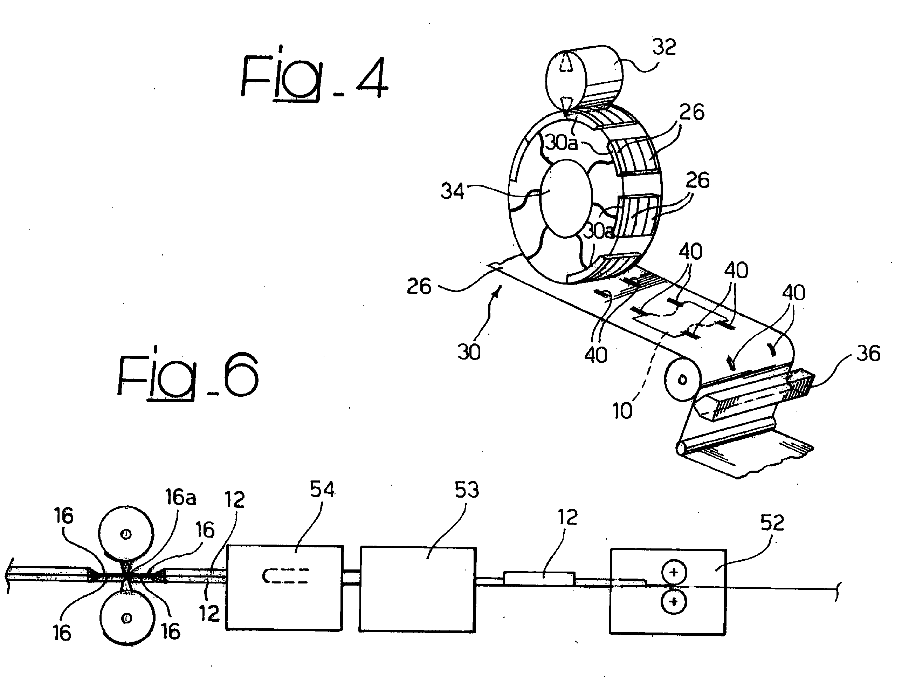

As may be appreciated more fully from the view of Figure 2 (and also from the view of Figure 7, which illustrates a chain of products 10 being processed), in the product 10 illustrated herein the central body 12 has two (front and rear) end parts, each with two opposite side edges 12a, from which there extend, on either side of the central body 12, the elasticated panels (or lugs) 16 that complete the central body 12, conferring on the product 10 as a whole (viewed in an open and extended condition) the typical hourglass configuration.

Each of the two ends, front and rear, of the central body 12 likewise has a respective terminal edge 12b substantially coinciding with the terminal end of the insert 14. Along the two terminal edges 12b (and along the homologous external margins 16) there extend two elasticated bands 18 that occupy the end margins (one front and one rear, in the position in which the product is worn) of the product itself.

It is emphasized that the terms "front" and "rear" are used herein only to distinguish the two ends from one another and hence does not have any specific importance as regards the mode in which the product is finally worn.

Furthermore, it is emphasized once again that the representation of the product 10 provided herein is intentionally schematic, precisely to facilitate an understanding of the solution described herein, which can be applied to a wide variety of possible types of embodiment of the product 10.

This applies, in particular but not exclusively, to the relative placing of the side edge 12a and terminal edge 12b of each of the ends of the central body 12 and of the elements (panels 16, elasticated band 18) associated thereto.

It will be understood, for example, in an evident way, that the proximal margin of each panel 16 can extend either slightly within, or in exact coincidence with, or again slightly on the outside of the respective side edge 12a, at the same time safeguarding in any case the characteristic according to which the two elasticated panels 16 extend from one and the other of the side edges 12a.

In an altogether similar way, the internal margin of the elasticated band 18 can extend either slightly within, or in exact coincidence with, or again slightly on the outside of the respective side edge 12b, at the same time safeguarding in any case the characteristic according to which the elasticated band 18 extends adjacent to the aforesaid terminal edge 12b and to the two elasticated panels 16.

The criteria of fabrication of the various component parts described previously are to be deemed in themselves widely known in the art and hence such as not to require a detailed description herein.

In particular, the side panels or patches 16 can be obtained according to the criteria described in greater detail in WO-A-01/91666 and WO-A-01/92013. This also regards the possibility of bestowing upon the panels 10 the characteristics of breathability, with the formation of openings for transpiration, at the same time limiting application of the elastic panels 16 only to the side areas (i.e., to the so-called "ears" of the product 10) and conserving the presence of the elasticated band (based on threads of Lycra or rubber) 18 at the end margins of the product.

It will thus be appreciated that the product 10 illustrated in the figures combines within it the characteristics of good fit linked to the presence of the elasticated panels 16 (a characteristic that up to now has principally been associated to the machine-direction technology), preserving, however, the presence of the elastic waist band 18 (a prerogative that is typical of products made using cross-direction technology) . The above is obtained at the same time, however, preventing the central parts of the so-called chassis of the product, on which the absorbent insert 14 is fixed usually by adhesive means, from being involved in an undesired action of elastication.

It will be appreciated, in particular, that the latter result is here achieved by preventing elastic elements from being located in said areas and not, as instead occurs in certain solutions adopted in the prior art, by providing elastic elements that are subsequently in some way neutralized or inactivated with operations of cutting which are liable to give rise to adverse effects either in structural terms or as regards the aesthetic appearance of the product.

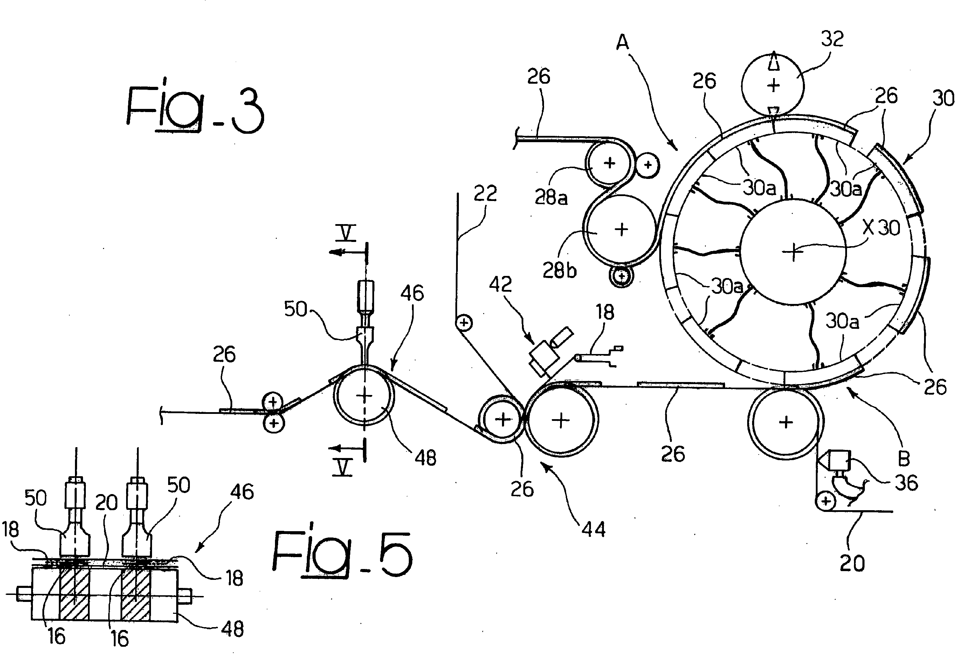

Figures 3 to 6 illustrate the salient characteristics of an apparatus that can be used for making the product illustrated in Figures 1 and 2.

Once again, it is emphasized that the elements of the apparatus represented in Figures 3 to 6 are basically designed for formation and application of the panels or patches 16 and of the end elasticated bands 18. Otherwise, with the exception of what is said specifically hereinafter, it is to be assumed that the remaining parts of the product 10 (for example, the absorbent insert 14 and, more in general, the entire central body 12) are obtained according to criteria in themselves known in the art and hence such as not to require any detailed description herein.

In particular, in the example of embodiment illustrated herein (which, it is emphasized, is just one such example, and is thus not to be interpreted as in any way limiting the scope of the invention) both the elastic panels or patches 16 and the end elasticated bands 18 are formed by resorting to a sandwich structure comprising:
- a central layer having elastic characteristics; and
- two outer layers typically constituted by a laminar material and in a particularly preferred way by a laminar material of the type commonly referred to as non-woven fabric.

In particular, the two layers in question are designed to constitute, respectively, the so-called backsheet or outer sheet of the product 10 and a covering layer applied on the backsheet with the interposition of the elastic elements.

Indicatively (and once again without this implying any limitation of the scope of the invention), the materials used for making the backsheet and the covering layer may be constituted, for example, by non-woven materials such as 18-g/m² microdenier spun bonded polypropylene.

With reference to the general diagram of Figure 3, it will be assumed that the layers of material in question, designated by 20 (backsheet) and 22 (covering layer) are fed in the direction of the parts of apparatus visible in Figure 3, from supply reels, which are not illustrated in the plate of drawings but are of a known type and which are set, respectively, on the right of Figure 3 and at the top with respect to said figure.

In a substantially similar way, the reference number 26 designates an elastic material that is to be used for making the panels or patches 16. Said material is wound (from left to right as viewed in Figure 3) off a respective supply reel, which is not visible in the plate of drawings either.

The elastic material 26 (which may be constituted, for example, by the weblike material marketed under the trade name CEX824 by the company Tredegar) is supplied "captured" between layers of polymer, i.e., in a rigid state designed to simplify the process of unwinding and of supply of the material up to the point of its application on the product. It is usually a material provided for applications in which the elastic characteristics of the material appear in the direction coinciding with the direction of delivery of the elastic material itself and the direction of feeding of the machine.

Immediately prior to application, the elastic material 26 must be activated, i.e., rendered such as to be able to perform its function of elasticated layer. For this purpose, it is necessary to proceed by stretching the weblike material 26 so as to tear the layers of polymer that imprison the elastic material itself in order to keep it fixed in a rigid state.

This result is typically obtained by causing the weblike material 26 to pass through a pair of motor-driven rollers 28a and 28b, which are able to perform both the function of feed and the function of activation of the weblike material 26.

As has been said, activation is obtained by tearing the layer of polymers that imprison the elastic material proper. This tearing action is performed by stretching and then releasing the web of material by suitably adjusting the speed of the rollers 28a and 28b. In particular, the approach is to cause the (peripheral) speed of the roller 28b to be higher (for example, three to five times higher) than the speed of the roller 28a. Usually, the distance between the two motor-driven rollers 28a and 28b is adjusted in such a way that it does not have an excessive value (for example, not greater than 50 mm) so as to guarantee a constant and homogeneous deformation throughout the development of the material.

The weblike material 26 thus activated is fed on the periphery of a distributing wheel 30 preferably configured in the form of a so-called "repitch" wheel.

Devices of this kind are known and used extensively in the industry of hygienic-sanitary products. Basically, the wheel 30 is constituted by a set of segments 30a (commonly referred to as "shoes") which are able to orbit about a central axis X30 with the capacity of varying selectively their distance between a loading region A, where the segments 30a of the periphery of the wheel 30 are strictly adjacent to one another, and an unloading region, designated by B, in which the segments 30a themselves are set with respect to one another at a distance determined selectively (according to criteria in themselves known).

Between the loading region A and the unloading region B there acts a cutter 32 (this is typically a rotary blade or a "hot-wire" cutter or cutting unit).

Hot-wire cutting can be adopted in the case where heat-meltable materials are used. The main advantage linked to hot-wire cutting lies in the fact that only the wire touches the material being cut. As compared to traditional cutting systems (for example, systems using a rotary blade), it is thus possible to eliminate completely the forces of cutting and, consequently, have a lighter mechanical structure. Furthermore, the hot-wire-cutting technique requires levels of precision of relative positioning of the parts that are less stringent than those of traditional cutting techniques, in particular in the case where the aim is to cause one and the same blade to be able to cut against a number of counterblades.

The result that can be achieved via the action of the repitch wheel 30 is more clearly represented in the perspective view of Figure 4. In said perspective view, it may be noted that the web 26, which is initially fed in the direction of the wheel 30 in the form of a continuous element, is segmented by the cutter 32 into a number of lengths, each length (or, more precisely - for reasons that will emerge more clearly in what follows -, each pair of lengths) being supported by a respective segment or shoe 30a of the peripheral wall of the wheel 30. The lengths of web 26 formed as a result of the action of the cutter 32 thus set themselves apart from one another reaching a relative parking position identified by the maximum value of the travel of relative recession of the segments or shoes 30a of the repitch wheel 30.

The lengths of web 26 are withheld on the shoes 30a of the wheel 30 by resorting (also here according to known criteria) to a suction-pressure effect ensured by a line or manifold of subatmospheric pressure designated by 34.

As has already been mentioned previously, the weblike material 26 is in actual fact fed in the form of two parallel webs, which are designed to form respective lengths of web (and, consequently, respective elasticated panels 16) located at either end of the products.

In particular, in the view of Figure 4, the web 20 constituting the so-called backsheet may be seen as it advances from the bottom right. In the movement of advance towards the wheel 30, the web 20 encounters a station for application of glue, designated by 36, which applies small areas of glue 40 on the backsheet 20, which are arranged according to a geometry substantially resembling the geometry with which the elastic panels 16 of the end product are located.

For a better illustration of this concept, Figure 4 represents with a dashed line the profile of one of the products 10 that it is intended to obtain. Of course, the representation of the profile with a dashed line in said part of Figure 4 has a purely explanatory character.

The lengths of web 26 supported by the segments or shoes 30a of the wheel 30 are thus applied in positions corresponding to the small adhesive areas 40 made on the backsheet 20.

The backsheet 20, thus equipped with the elastic elements 26, advances towards a further processing station 42, which applies on the side edges of the web of backsheet 20 Lycra or rubber elastics designed to form the waist-band elastication 18 of the product (see, in particular, the view of the distended product illustrated in Figure 2).

Typically, the elastics 18 are applied on the backsheet 20 by gluing, for example using a spray glue. Of course, it is possible to resort to other technological solutions, well known to persons skilled in the branch.

After receiving the elastics 18 (which, it will be appreciated, are applied by operating in the direction of feeding of the machine), the backsheet 20, which at this point is equipped with the various elastic elements designed to be associated thereto, passes into a coupling station 44 to obtain (according to known criteria) pairing with the covering layer 22 so as to complete the sandwich formation described previously.

Having in this way completed the structure of the so-called chassis of the product, the web of backsheet, provided with elasticated panels 16 and elasticated bands 18 is made to pass under an ultrasound-sealing system 46, which joins together the various layers thus also obtaining, in the areas corresponding to the panels 16, ventilation openings that bestow characteristics of breathability upon the panels.

The diagram of Figure 5 is a schematic representation of the modality of execution of said operation performed by passing the backsheet 20 with the elastic elements 16 and 18 applied thereon over a roller 48 functioning as the so-called "anvil" of an ultrasound-sealing system of which the so-called "sonotrodes" 50 are visible. The sonotrodes in question number two (or form two groups) and are designed to carry out a sealing operation at what will form the front region and the rear region of the product. Further details regarding execution of these operations can be inferred, for example, from WO-A-01/91666 and WO-A-01/92013 already cited previously.

The web provided with the elastic elements 16 and 18 can then be sent on to a coupling station 52 (Figure 6, which constitutes an ideal continuation of Figure 3), where applied on the aforesaid web, operating crosswise (and usually making the connection adhesively, according to known criteria), are the central bodies 12.

The operating situation can be appreciated more fully from the perspective view provided in Figure 7. From this view it will also be noted that, in a currently preferred embodiment, the overall width of the web of backsheet 20 is chosen so as to be sufficiently longer than the overall length of the central body 12.

To be more precise, the overall width of the web of backsheet 20 is chosen so as to be longer than the distance that separate the outer margins, located at the opposite ends of the product, of the elasticated bands 18. This fact can immediately be appreciated in Figure 7 if it is noted that the web of backsheet 20 projects on either side of the web 22 that "covers" the elasticated bands 18.

In this way, in a folding station 53, it is possible to turn up the outer edges of the web of backsheet 20 towards the inside of the product, as is indicated by 200 in Figure 7, so as to "entrap" the terminal regions of the absorbent insert 14.

Next, in an purposely provided folding station 54, the web is folded according to a general V-shaped configuration in the longitudinal middle area.

This is followed, in a final station designated by 56, by closing of the end margins 16a of the panels 16 (and of the elasticated bands 18), with consequent closing of the product along the line of waist band and segmentation of the individual products 10 thus obtained.

The fact that the elasticated panels 16 and the elasticated band 18 are connected, at either end of the product 10, to homologous elements so as to reclose the product 10, bestows upon the product itself the character of a product of a training-pant type.

The latter operations described are obtained according to criteria widely known in the art and hence such as not to require any detailed description herein.

In particular, closing of the end margins 16a of the panels 16 and of the elasticated bands 18 along the line of waist band of the product can both assume the character of a stable seal and involve application (performed once again according to known criteria) of fastening elements of an openable and refastenable type, for example with the use of hook-and-loop elements of the Velcro type.

It will be appreciated that said operations are performed according to typical cross-direction modalities of fabrication, at the same time making it possible to arrive at an end product that has all the elements and the positive characteristics of the product obtained with the machine-direction technique.

Basically, the method described herein envisages feeding in a given direction a weblike element (the backsheet 20) having opposite side edges 12a, and applying on said weblike element, with a pitch of separation identified by the width of the product 10, patches of elastic material that can define the elasticated panels 16 which, at the corresponding end of said central body 12, each define a portion of the line of waist band of the product.

Then applied along at least one of the side edges of the web of backsheet 20 is, in the station 42, a continuous elasticating element designed to form an elasticating waist-band portion of the product. Applied then on the web of backsheet 20 are, in a position generically set between the aforesaid patches of elastic material, the central bodies 12 of the products 10 undergoing fabrication. It will be appreciated that said central bodies 12 are applied on the web of backsheet with their opposite ends aligned in a direction transverse to the direction of feed of the web of backsheet 20, i.e., operating in a cross-direction modality.

The weblike element thus formed is then segmented in a substantially median position between the central bodies 12 subsequently applied so as to form individual products 10. This occurs preferably after folding to form a V performed in the station 54 and after having closed the opposite side edges 16a of the elasticated panels 16 and of the elasticated waist bands 18 combined therewith, thus obtaining the products 10 in the form of closed pantlike products of the training-pant type.

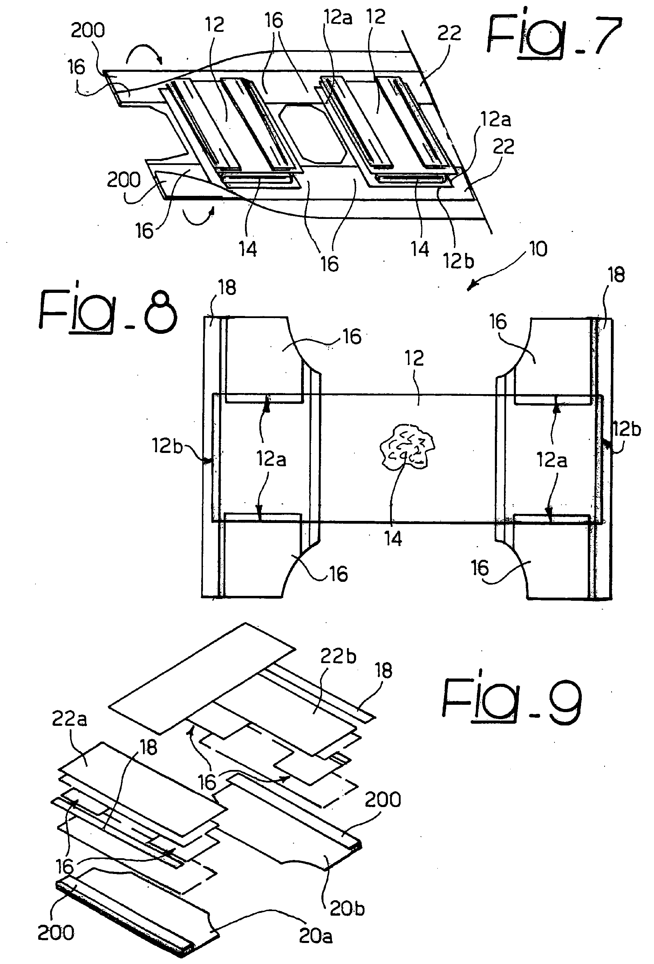

The mode of operation described enables use of a web of backsheet 20, which, instead of being a single piece, as schematically illustrated in the foregoing figures, is in actual fact constituted by two "subwebs" 20a and 20b separated from one another. Each of said subwebs is designed to form, in combination with the elastic patches of the panels 16 and with the elasticated waist band 18, and with a corresponding covering subweb 22a and 22b, a respective end of the chassis of the product, as is schematically illustrated in Figures 8 and 9.

By adopting the solution represented in Figures 8 and 9, instead of using a single chassis, a chassis is used that is made up of two parts, a front one and a rear one, which are held together by the central body 12, which extends like a bridge between the two parts in question.

The advantages potentially linked to the solution illustrated in Figures 8 and 9 are multiple.

In the first place, this solution enables a reduction in the cost of the end product in so far as a smaller amount of material is used for making the chassis. Furthermore, the solution represented in Figures 7 and 8 enables the operation of "change of format", i.e., of selective variation of the dimensions of the end product, to be carried out very rapidly. To achieve this result, it is in fact sufficient to space the two portions 20a, 20b of the web of backsheet in a different way to obtain different lengths of the product, at the same time preserving the same resultant type of product.

Figure 9 highlights the fact that, even in the case of this variant embodiment, there exists the possibility of turning inwards the outer margins 200 of the web of backsheet (20a and 20b, in this case), so as to "entrap" the terminal regions of the absorbent insert 14.

Of course, without prejudice to the principle of the invention, the details of construction and the embodiments may vary widely with respect to what is described and illustrated herein, without thereby departing from the scope of the present invention.

In particular, persons skilled in the sector will appreciate that, even though the solution described herein has been developed paying particular attention to its possible application to products of a training-pant type (i.e., products designed to be sold in a pant-like configuration), it can also be applied to diapers of a traditional type.

Furthermore, the solution described herein as applied at both ends (front and rear) of the product 10, could in actual fact be applied also at just one of said ends, making the opposite end of the product according to any traditional solution.

Also as regards the process of fabrication, the invention contemplates the possibility of resorting to a wide range of variants as compared to the examples of embodiment illustrated herein. For example, above all when the cross-direction mode of operation is adopted, it may be advantageous to apply the elastic panels 16 in an intermittent way, i.e., causing the adjacent elastic panels which are designed to form part of different end products to be right from the start elements distinct from one another and to be separated only at the moment in which the respective products are separated from the chain or sequence in which they have been fabricated. This mode of operation enables prevention of the need to perform the corresponding operations of sealing and cutting over an area comprising the material of the elastic panel. In this way, it is possible to obtain the sealing and cutting area without elastic panel, thus avoiding problems linked to the tightness of the seal which may arise, in certain cases, on account of the choice of the elastic material.

In more recent times, elastic materials have been developed that have a mesh-like structure, which are excellent as regards breathability. The invention comprises the possible adoption of all materials that can be used for obtaining the panels 16, such as, precisely, also the aforesaid elastic materials having a mesh-like structure.

## Claims

1. An absorbent sanitary product (10) wearable as a pantlike garment, comprising:
- a central body (12) comprising an at least partially absorbent insert (14), defining the crotch portion of the product (10); said central body (12) having opposite ends, each with two opposite side edges (12a) and a terminal edge (12b); and
- at at least one of said opposite ends of said central body (12):
- two elasticated panels (16) extending from either of said two side edges (12a) so as to define, at the corresponding ends of said central body (12), a respective portion of the line of waist band of the product (10); and
- an elasticated band (18) extending adjacent to said terminal edge (12b) and to said two elasticated panels (16) so as to define, at each corresponding end of said central body (12), a respective elasticating portion of the line of waist band (18) of the product (10).

2. The product according to Claim 1, **characterized in that** said opposite ends of said central body (12) are substantially free from elastication.

3. The product according to Claim 1 or Claim 2, **characterized in that** said elastic panels (16) are provided with openings for breathing.

4. The product according to any one of the preceding claims, **characterized in that** said elasticated panels (16) have a stratified structure with a layer of elastic material (26) coupled to at least one layer of non-elastic material (20).

5. The product according to Claim 4, **characterized in that** said elasticated panels (16) have a sandwich structure with said layer of elastic material (26) set between two layers (20, 22) of non-elastic material.

6. The product according to Claim 4 or Claim 5, **characterized in that** said at least one layer of non-elastic material (20, 22) is constituted by non-woven fabric.

7. The product according to any one of the preceding Claims, **characterized in that** said elasticated band (18) has a stratified structure with at least one elastic body applied on a layer of non-elastic material (20) .

8. The product according to Claim 7, **characterized in that** said elasticated band (18) has a sandwich structure with said elastic body set between two layers (20, 22) of non-elastic material.

9. The product according to Claim 7 or Claim 8, **characterized in that** said at least one layer of non-elastic material (20, 22) is constituted by non-woven fabric.

10. The product according to Claim 4 or Claim 7, **characterized in that** said at least one layer of non-elastic material (20) is common both to said elasticated panels (16) and to said elasticated band (18).

11. The product according to any one of Claim 4, Claim 7 or Claim 10, **characterized in that** said at least one layer of non-elastic material (20) constitutes, at least in part, the backsheet of the product (10).

12. The product according to Claim 11, **characterized in that** said at least one layer of non-elastic material (20) extends continuously between said opposite ends of said central body (12).

13. The product according to Claim 11, **characterized in that** said at least one layer of non-elastic material (20) is constituted by two portions (20a, 20b), separated from one another and located at either of said two opposite ends of said central body (12).

14. The product according to any one of Claim 7 or Claim 8, **characterized in that** said at least one layer of non-elastic material (20) has a margin turned up (200) against the product (10) at said terminal edge (12b) in a relationship of trapping of said at least partially absorbent insert (14).

15. The product according to any one of the preceding claims, **characterized in that** said elasticated panels (16) and said elasticated band (18) are connected to homologous elements so as to enable refastening of the product (10) bestowing thereon the character of product of a "training pant" type.

16. The product according to Claim 15, **characterized in that** said elasticated panels (16) and said elasticated band (18) are connected to homologous elements via elements of an openable and refastenable type.

17. A method for making a product according to any one of Claims 1 to 16, **characterized in that** it comprises the operations of:
- feeding in a given direction a weblike element (20) having opposite side edges (12a);
- applying, on said weblike element (20) and with a pitch of separation identified by the width of said product, patches of elastic material (26) that are able to define said elasticated panels (16);
- applying, along at least one of said side edges of said weblike element (20), a continuous elasticating element that is to form said elasticated band (18);
- applying on said weblike element (20), in a position generically set between said elastic patches (16) subsequently applied on said weblike element (20), the central bodies (12) of respective said products (10); said central bodies (12) being applied on said weblike element (20) with said opposite ends aligned in a direction transverse to said given direction of feed of said weblike element (20); and
- cutting said weblike element (20) in a substantially median position between central bodies (12) subsequently applied on said weblike element (20) so as to form individual products (10).

18. The method according to Claim 17, **characterized in that** it comprises the operation of providing, as said weblike element (20), a single weblike element.

19. The method according to Claim 17, **characterized in that** it comprises the operation of providing, as weblike element (20), a pair of weblike formations (20a, 20b) set at a distance from one another, then connecting said weblike formations separated from one another to said central bodies (12) of respective said products (10) arranged so as to connect by bridging said weblike formations (20a, 20b) set at a distance from one another.

20. The method according to any one of Claims 17 to 19, **characterized in that** it comprises, after the operation of applying said central bodies (12), the operation of folding (54) said weblike element (20) to form a V so as to bring said opposite side edges (12a) into a condition where they are substantially set on top of one another.

21. The method according to Claim 20, **characterized in that** it comprises the operation of connecting to one another said opposite side edges (12a) set on top of one another at the outer ends (16a) of said elasticated panels (16) and of said elasticated band (18) thus obtaining said products (10) in the form of closed-pant products.

22. The method according to any one of Claims 17 to 21, **characterized in that** it comprises the operation of subjecting said elasticated panels (16) to an operation of formation of openings for breathing.

23. The method according to any one of the preceding Claims 17 to 22, **characterized in that** it comprises the operation of making said elasticated panels (16) with a stratified structure with a layer of elastic material (26) paired with at least one layer of non-elastic material (20).

24. The method according to Claim 23, **characterized in that** it comprises the operation of making said elasticated panels (16) with a sandwich structure with said layer of elastic material (24) set between two layers (20, 22) of non-elastic material.

25. The method according to Claim 23 or Claim 24, **characterized in that** said at least one layer of non-elastic material (20, 22) is constituted by non-woven fabric.

26. The method according to any one of the preceding Claims 17 to 25, **characterized in that** it comprises the operation of making said elasticated band (18) with a stratified structure with at least one elastic body applied on a layer of non-elastic material (20) .

27. The method according to Claim 26, **characterized in that** it comprises the operation of making said elasticated band (18) with a sandwich structure with said elastic body set between two layers (20, 22) of non-elastic material.

28. The method according to Claim 26 or Claim 27, **characterized in that** said at least one layer of non-elastic material (20, 22) is constituted by non-woven fabric.

29. The method according to Claim 23 or Claim 26, **characterized in that** said at least one layer of non-elastic material (20) is common both to said elasticated panels (16) and to said elasticated band (18) .

30. The method according to any one of Claim 23, Claim 26 or Claim 29, **characterized in that** comprises the operation of making at least in part the backsheet of the product (10) with said at least one layer of non-elastic material (20).

31. The method according to Claim 30, **characterized in that** it comprises the operation of extending said at least one layer of non-elastic material (20) continuously between said opposite ends of said central body (12) .

32. The method according to Claim 30, **characterized in that** it comprises the operation of using, as said at least one layer of non-elastic material (20), two weblike portions (20a, 20b) separated from one another and located at either of said opposite ends of said central body (12).

33. The method according to Claim 26 or Claim 27, **characterized in that** it comprises the operation of turning up (200) said at least one layer of non-elastic material (20) against the product (10) at said terminal edge (12b) in a relationship of trapping of said at least partially absorbent insert (14).
